(19) Europäisches Patentamt
European Patent Office
Office européen des brevets

(11) Publication number: **0 372 998**
**A2**

(12) # EUROPEAN PATENT APPLICATION

(21) Application number: **89312839.7**

(51) Int. Cl.[5]: **A61K 31/44, A61K 31/00**

(22) Date of filing: **08.12.89**

The title of the invention has been amended (Guidelines for Examination in the EPO, A-III, 7.3).

(30) Priority: **08.12.88 GB 8828679**

(43) Date of publication of application: **13.06.90 Bulletin 90/24**

(84) Designated Contracting States: **AT BE CH DE ES FR GB GR IT LI LU NL SE**

(71) Applicant: **BEECHAM GROUP PLC**
**Beecham House Great West Road**
**Brentford Middlesex TW8 9BD(GB)**

(72) Inventor: **Hamilton, Thomas Conway**
**Beecham Pharmaceuticals**
**Medicinal Res. Centre Coldharbour Road**
**Pinnacles**
**Fourth Avenue Harlow Essex CM19 5AD(GB)**

(74) Representative: **Jones, Pauline et al**
**Beecham Pharmaceuticals Patents & Trade Marks Dept. Great Burgh Yew Tree Bottom Road**
**Epsom Surrey KT18 5XQ(GB)**

(54) **Use of benzopyran derivatives in the treatment of epilepsy.**

(57) A method for the treatment of epilepsy comprising the administration of a potassium channel activator, such as (-)-6-cyano-3,4-dihydro-2,2-dimethyl-trans-4-(2-oxo-1-pyrrolidinyl)-2H-benzo[b]pyran-3-ol.

EP 0 372 998 A2

## NOVEL TREATMENT

The present invention relates to a method for the treatment and/or prophylaxis of epilepsy.

EP-A-76075, 91748, 93535, 95316, 107423, 120426, 120427, 126311, 126350, 126367, 138134, 168619, 205292, 214818, 250077, 321175, WO 89/05808 and European Patent Application No. 89309272.6 (Beecham Group p.l.c.) describe classes of compounds which are believed to be potassium channel activator antihypertensive agents.

U.K. Patent No. 1489879 discloses the hypotensive compound, N″-cyano-N-4-pyridyl-N′-1,2,2-trimethylpropylguanidine (pinacidil) and, in Example 47, a process by which it can be prepared. In "Drugs of the Future" Vol. VI(3), 149, 1981, pinacidil is described as a vasodilator. It is now known that pinacidil is a potassium channel activator.

EP-A-314446 (American Home Products Corporation), EP-A-296975 and 312432 (Sanofi), EP-A-298452 (F. Hoffmann-La Roche and Co.), EP-A-273262, EP-A-308972 and 340718 (Merck Patent GmbH), EP-A-277611, EP-A-277612 and 337179 (Hoechst Aktiengesellschaft), EP-A-339562 (Yoshitomi Pharmaceutical Industries Ltd.), GB 2204868A (Sandoz Limited) and WO 89/07103 (Nissan Chemical Industries Ltd.) describe classes of benzopyran derivatives which are believed to be potassium channel activator antihypertensive agents.

It has now been discovered that compounds of these classes are of potential use as neuronal protective agents, such as anticonvulsants in the treatment of epilepsy.

Accordingly, the present invention provides a method for the treatment and/or prophylaxis of epilepsy in mammals, such as humans, which method comprises administering to the mammal in need of such treatment and/or prophylaxis an effective and/or prophylactic amount of a potassium channel activator, such as pinacidil or a compound of formula (I), or a pharmaceutically acceptable salt thereof:

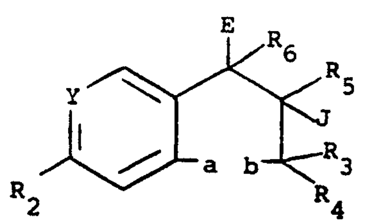

(I)

wherein

a and b together form an -O- linkage or (when E is of structure i) wherein X is O or S and $R_8$ is hydrogen or $C_{1-6}$ alkyl, $R_1$ and $R_2$ are not joined and J is hydrogen) a and b together form a $-CH_2-$ linkage or a bond;

either Y is N and $R_2$ is hydrogen; or

Y is C-$R_1$

wherein

either one of $R_1$ and $R_2$ is hydrogen and the other is nitro, cyano, halo, $CF_3$, formyl, aldoxime, $CF_3O$, $NO_2$-CH=CH-, NC-CH=CH-; a group $R_xX$-wherein $R_x$ is $C_{1-6}$ alkyl, aryl or heteroaryl either of which may be optionally substituted by one, two or three of $C_{1-4}$ alkyl, $C_{1-4}$ alkoxy, nitro, halo, $CF_3$ and cyano; and X is C=O, O.C=O, C=O.O, CHOH, SO, $SO_2$, O.SO, O.$SO_2$, CONH, O.CONH, O.$SO_2$NH, CO-CH=CH, C=NHOH, C=$NNH_2$; or a group $R_yR_zNZ$- wherein $R_y$ and $R_z$ are independently hydrogen or $C_{1-6}$ alkyl and Z is C=O, SO or $SO_2$; or a group $(R_wO)_2P(O)W$ wherein $R_w$ is hydrogen or $C_{1-6}$ alkyl and W is O or a bond; or

$R_1$ is a $C_{3-8}$ cycloalkyl group or a $C_{1-6}$ alkyl group optionally substituted by a group which is hydroxy, $C_{1-6}$ alkoxy, amino optionally substituted by one or two $C_{1-6}$ alkyl groups, $C_{1-7}$ alkanoylamino, $C_{3-8}$ cycloalkyloxy or $C_{3-8}$ cycloalkylamino; and $R_2$ is hydrogen; or

one of $R_1$ and $R_2$ is nitro, cyano or $C_{1-3}$ alkylcarbonyl and the other is a different group selected from nitro, cyano, halo, $C_{1-3}$ alkylcarbonyl, methoxy or amino optionally substituted by one or two $C_{1-6}$ alkyl or by $C_{2-7}$ alkanoyl; or

$R_1$ and $R_2$ together with the carbon atoms to which they are attached, form 2,1,3-oxadiazole;

either one of $R_3$ and $R_4$ is hydrogen or $C_{1-4}$ alkyl and the other is $C_{1-4}$ alkyl; or

$R_3$ and $R_4$ together are $C_{2-5}$ polymethylene;

either $R_5$ is hydrogen, hydroxy, $C_{1-6}$ alkoxy, $C_{1-7}$ acyloxy or $ONO_2$; and

$R_6$ is hydrogen; or
$R_5$ and $R_6$ together are a bond;
J is hydrogen or $C_{1-6}$ alkyl;
E is a group of structure i):

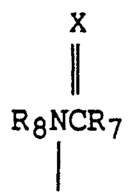

i)

wherein
either X is oxygen or sulphur; and
$R_7$ is hydrogen, $C_{1-6}$ alkyl optionally substituted by hydroxy, $C_{1-6}$ alkoxy, $C_{1-6}$ alkoxycarbonyl or carboxy, $C_{1-6}$ alkyl substituted by halogen, or $C_{2-6}$ alkenyl; aryl or heteroaryl either being optionally substituted by one or more groups or atoms selected from the class of $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and
$R_8$ is hydrogen, $C_{1-6}$ alkyl, or a group $OR_p$ or $NHCOR_q$ wherein $R_p$ is hydrogen, $C_{1-6}$ alkyl, aralkyl, $C_{1-7}$ alkanoyl or aroyl and $R_q$ is as defined above for $R_7$; or
$R_7$ and $R_8$ are joined together to form $C_{3-4}$ polymethylene optionally substituted by one or two $C_{1-6}$ alkyl groups or $-CH_2-(CH_2)_n-Z-(CH_2)_m-$wherein m and n are integers 0 to 2 such that m+n is 1 or 2 and Z is oxygen, sulphur or $NR_9$ wherein $R_9$ is hydrogen, $C_{1-9}$ alkyl, $C_{2-7}$ alkanoyl, phenyl $C_{1-4}$ alkyl, naphthylcarbonyl, phenylcarbonyl or benzylcarbonyl optionally substituted in the phenyl or naphthyl ring by one or two $C_{1-6}$ alkyl, $C_{1-6}$ alkoxy or halogen; mono- or bi-cyclic heteroarylcarbonyl; or $R_7$ and $R_8$ are joined to form $-B^1=B^2-B^3=B^4-$ wherein one of $B^1$ to $B^4$ is $CR_r$ or N and the other three are $CR_r$ wherein $R_r$ is hydrogen or $C_{1-6}$ alkyl;
or X is N-CN, $N-NO_2$, $N-COR_{10}$ or $N-SO_2R_{10}$ wherein $R_{10}$ is $C_{1-3}$ alkyl, $NH_2$, $NH(C_{1-3}$ alkyl), $CF_3$ or phenyl optionally substituted as defined for $R_x$; and
$R_7$ is $NHR_{11}$ wherein $R_{11}$ is hydrogen, $C_{1-6}$ alkyl, $C_{3-6}$ cycloalkyl, $C_{2-6}$ alkenyl or $C_{2-6}$ alkynyl; and
$R_8$ is hydrogen or $C_{1-6}$ alkyl; or
$R_7$ and $R_8$ together are $C_{2-4}$ polymethylene;
or E is a group of structure ii):

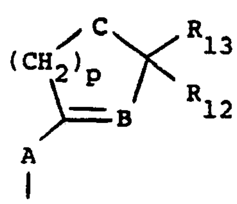

ii)

wherein
A is O or $NR_{14}$ wherein $R_{14}$ is hydrogen, $C_{1-4}$ alkyl, formyl, acetyl or hydroxymethyl;
B is N or $CR_{15}$ wherein $R_{15}$ is hydrogen, halogen, formyl or hydroxymethyl;
C is $CH_2$, O, S, CH-Halogen, amino or $C_{1-6}$ alkylamino; p is 1, 2 or 3; and
$R_{12}$ and $R_{13}$ are independently hydrogen or methyl or together are oxo- or thio-;
or E is tetrahydroisoquinolinone, 2,3-dihydro-1H-isoindol-1-one, 2-pyridine N-oxide, 2-hydroxyphenyl or 2-hydroxypyridine (all four possible isomers);
the E moiety being trans to the $R_5$ group when $R_5$ is other than hydrogen or a bond.

Suitable and preferred values for the variable groups or atoms in formula (I) are as described for the corresponding variables in EP-A-76075, 91748, 93535, 95316, 107423, 120426, 120427, 126311, 126350, 126367, 138134, 168619, 205292, 214818, 250077 and 321175; and also the patent references hereinbefore referred to. Corresponding United States Patent references include U.S. Patent No's. 4446113, 4542149, 4510152, 4481214, 4496565, 4555509, 4610992, 4571406, 4629734, 4575511, 4800212, 4677116 and 4812459.

All $C_{1-6}$ alkyl or alkyl containing groups in formula (I) are preferably selected from methyl, ethyl, n- and iso-propyl, n-, iso-, sec- and tert-butyl.

$C_{3-8}$ cycloalkyl groups are cyclopropyl, cyclobutyl, cyclopentyl, cyclohexyl, cycloheptyl and cyclooctyl.

Aryl includes phenyl and naphthyl.

Heteroaryl includes a 5- or 6- membered monocyclic or 9- or 10- membered bicyclic of which 5- or 6-memebered monocyclic heteroaryl is preferred. In addition, 5- or 6-membered monocyclic or 9- or 10-membered bicyclic heteroaryl preferably contains one, two or three heteroatoms which are selected from the class of oxygen, nitrogen and sulphur and which, in the case of there being more than one heteroatom, are the same or different. Examples of 5- or 6-membered monocyclic heteroaryl containing one, two or three heteroatoms which are selected from the class of oxygen, nitrogen and sulphur include furyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2- and 3-furyl, 2- and 3-pyrryl, 2- and 3-thienyl, and 2-, 3-and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazolyl. Preferred examples of such groups include 2- and 3-benzofuryl, 2- and 3-benzothienyl, and 2- and 3-indolyl, and 2- and 3-quinolyl.

Suitable examples of groups or atoms for optional substitution of aryl and heteroaryl include one, two or three substituents independently selected from $C_{1-4}$ alkyl, $C_{1-4}$ alkoxy, halo (such as fluoro, chloro, bromo), hydroxy, nitro and cyano.

Acyl groups are preferably carboxylic acyl, usually alkanoyl.

There is a group of compounds within formula (I) wherein E is $R_8NCXR_7$ wherein X is oxygen or sulphur, J is hydrogen, and:-

either one of $R_1$ and $R_2$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkylthiocarbonyloxy, 1-mercapto $C_{2-7}$ alkyl, formyl, or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino, $C_{1-6}$ alkoxysulphinylamino or $C_{1-6}$ alkoxysulphonylamino, or ethylenyl terminally substituted by $C_{1-6}$ alkylcarbonyl, nitro or cyano, or $-C(C_{1-6}\ alkyl)NOH$ or $-C(C_{1-6}\ alkyl)NNH_2$; and

$R_7$ is hydrogen, $C_{1-6}$ alkyl optionally substituted by hydroxy, $C_{1-6}$ alkoxy, $C_{1-6}$ alkoxycarbonyl or carboxy, $C_{1-6}$ alkyl substituted by halogen, or $C_{2-6}$ alkenyl; aryl or heteroaryl either being optionally substituted by one or more groups or atoms selected from the class of $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and

$R_8$ is hydrogen or $C_{1-6}$ alkyl; or

$R_7$ and $R_8$ are joined together to form $C_{3-4}$ polymethylene or $-CH_2-(CH_2)_n-Z-(CH_2)_m-$ wherein m and n are integers 0 to 2 such that m+n is 1 or 2 and Z is oxygen, sulphur or $NR_9$ wherein $R_9$ is hydrogen, $C_{1-9}$ alkyl, $C_{2-7}$ alkanoyl, phenyl $C_{1-4}$ alkyl, naphthylcarbonyl, phenylcarbonyl or benzylcarbonyl optionally substituted in the phenyl or naphthyl ring by one or two $C_{1-6}$ alkyl, $C_{1-6}$ alkoxy or halogen; mono- or bi-cyclic heteroarylcarbonyl;

and the remaining variables are as defined in formula (I).

A particularly preferred compound of formula (I) is the compound of Example 1 of EP-A-76075 and United States Patent No. 4446113, (±)-6-cyano-3,4-dihydro-2,2-dimethyl-trans-4-(2-oxo-1-pyrrolidinyl)-2H-benzo[b]pyran-3-ol, also known as cromakalim; and its (-)-enantiomer, also known as BRL 38227, disclosed in EP-A-120428.

Examples of pharmaceutically acceptable salts are as described in the aforementioned European Patent references, the subject matter of which are incorporated herein by reference.

References to a potassium channel activator, including pinacidil or a compound of formula (I) and salts thereof, include solvates such as hydrates.

Potassium channel activators may be identified by standard methods, such as those described in EP-A-176689.

The compounds of formula (I), and salts thereof may be prepared as described in the aforementioned Patent Publications/References.

Preferably, a compound of formula (I) is in substantially pure pharmaceutically acceptable form.

Examples of the compounds of formula (I) include the examples described in the aforementioned Patent Publications/References.

It will be appreciated that the benzopyran compounds of formula (I) wherein $R_5$ is hydroxy, alkoxy, acyloxy or $ONO_2$, and/or wherein J is $C_{1-6}$ alkyl, have an asymmetric centre at the 3- and 4- carbon atoms, and are capable of existing in the (3R, 4S) and (3S, 4R) forms. The invention extends to each of these forms including racemates. The (3S, 4R) form is believed to be preferred when J is hydrogen.

The administration of the potassium channel activator may be by way of oral, sublingual, transdermal or parenteral administration.

An amount effective to treat the disorders hereinbefore described depends on the usual factors such as the nature and severity of the disorders being treated and the weight of the mammal. However, a unit dose will normally contain 0.1 to 50 mg for example 0.5 to 10 mg, of the potassium channel activator, such as pinacidil or the compound of formula (I) or a pharmaceutically acceptable salt thereof. Unit doses will normally be administered once or more than once a day, for example 2, 3, or 4 times a day, more usually 1 to 3 times a day, such that the total daily dose is normally in the range, for a 70 kg adult of 0.1 to 50 mg, for example 0.5 to 10 mg, that is in the range of approximately 0.001 to 1 mg/kg/day, more usually 0.005 to 0.2 mg/kg/day.

For oral or parenteral administration, it is greatly preferred that the potassium channel activator is administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling or tabletting. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

As is common practice, the compositions will usually be accompanied by written or printed directions for use in the treatment concerned.

The present invention also provides a potassium channel activator, such as pinacidil or a compound of formula (I) or a pharmaceutically acceptable salt thereof for the manufacture of a medicament for use in the

treatment and/or prophylaxis of epilepsy. Such treatment and/or prophylaxis may be carried out as hereinbefore described.

The present invention further provides a pharmaceutical composition for use in the treatment and/or prophylaxis of epilepsy which comprises a potassium channel activator, such as pinacidil or a compound of formula (I) or a pharmaceutically acceptable salt thereof and a pharmaceutically acceptable carrier.

Such compositions may be prepared in the manner as hereinbefore described.

The following pharmacological data illustrate the activity of potassium channel activators in tests which are indicative of compounds of potential use in the treatment of epilepsy.

## Pharmacological Data

An example of a model of epilspsy is the prevention of seizures induced by the bee venom peptide MCD (mast cell degranulating peptide) in rats, as described by G. Gandolfo et. al. Europ. J. Pharmacol., 1989, 159, 329. The method and results obtained therein are outlined below:-

### Method

Under anaesthesia (60 mg/kg i.p. of penthiobarbital), male Wistar rats are implanted with a bevel-edged cannula directed toward the lateral ventricle and with a deep multipolar electrode in the dorsal hippocampaus. Silver balls are placed on the frontal and occipital cortex. The electromyogram is recorded from the dorsal neck muscles. Postoperative care and recording procedures are carried out as described previously (Bidard et al., Brain Res. 418, 235, 1987). Recordings are performed on 3 groups of rats: (i) control rats receive an i.c.v. injection of MCD alone (10µl during 2 min. the injection needle remaining in the cannula two more minutes to avoid flow back of the toxin solution). (ii) sham rats receive an i.c.v. injection of vehicle (either 0.1-1% dimethylsulfoxide in isotonic saline solution or 0.7-7% v/v ethanol/saline solution) prior to MCD administration. (iii) the experimental group of rats receive an i.c.v. injection of the $K^+$ channel activator during the 15-20 min prior to MCD administration or during the MCD-induced seizures.

An i.c.v. injection of MCD induces long-lasting hippocampal theta rhythm at a dose of 0.05 nmol and seizures at a dose of 0.10 nmol or higher (see Bidard et al., 1987). MCD-induced seizures are quantified by their frequency of occurrence (table 1). In control and sham rats, the frequency of seizures increases with increasing doses of MCD.

The results are as shown in Table 1.

To simplify, only the mean frequency of the occurence of induced seizures during 10 minutes after the first generalised crisis are given, in $min^{-1}$. A value of 1 indicates continuous seizures. An absence of seizures is indicated by 0.

Complete or almost complete prevention of MCD-induced seizures (P<0.001) is observed after a previous injection of BRL 38227 (from a dose of 10 nmol).

Table 1

| n (no of rats) | | | MCD dose (nmol) | | | |
|---|---|---|---|---|---|---|
| | | | 0.10 | 0.15 | 0.30[a] | 0.45[a] |
| | | | Frequency of seizures ($min^{-1}$) | | | |
| Control | 5 | | 0.2 | 0.5 | 0.8 | 1 |
| | | Student's t-test | P<0.001 | | P<0.01 | NS |
| Sham | 6 | | 0.1 | 0.3 | | |
| | | Student's t-test | P<0.01 | | | |
| BRL 38227 | 6 | 100 nmol | 0 | 0 | 0 | 0 |
| | 6 | 10 nmol | 0 | 0 | 0 | 0[b] |
| | 2 | 1 nmol | 0 | 0 | 0.6 | |

[a] lethal doses [b] In one rat only a short lasting (<10s) crisis occurred without any following crisis at a dose of MCD 1.5 fold higher than the lethal one.

## Claims

1. Use of a potassium channel activator in the manufacture of a medicament for the treatment of epilepsy.

2. A use according to claim 1 wherein the potassium channel activator is pinacidil or a compound of formula (I), or a pharmaceutically acceptable salt thereof:

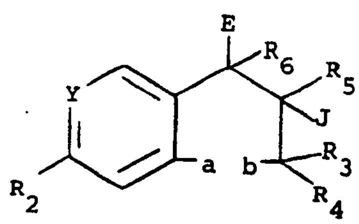

(I)

wherein

a and b together form an -O- linkage or (when E is of structure i) wherein X is O or S and $R_8$ is hydrogen or $C_{1-6}$ alkyl, $R_1$ and $R_2$ are not joined and J is hydrogen) a and b together form a $-CH_2-$ linkage or a bond;

either Y is N and $R_2$ is hydrogen; or

Y is C-$R_1$

wherein

either one of $R_1$ and $R_2$ is hydrogen and the other is nitro, cyano, halo, $CF_3$, formyl, aldoxime, $CF_3O$, $NO_2$-CH=CH-, NC-CH=CH-; a group $R_xX$-wherein $R_x$ is $C_{1-6}$ alkyl, aryl or heteroaryl either of which may be optionally substituted by one, two or three of $C_{1-4}$ alkyl, $C_{1-4}$ alkoxy, nitro, halo, $CF_3$ and cyano; and X is C=O, O.C=O, C=O.O, CHOH, SO, $SO_2$, O.SO, O.$SO_2$, CONH, O.CONH, O.$SO_2$NH, CO-CH=CH, C=NHOH, C=$NNH_2$; or a group $R_yR_zNZ$- wherein $R_y$ and $R_z$ are independently hydrogen or $C_{1-6}$ alkyl and Z is C=O, SO or $SO_2$; or a group $(R_wO)_2P(O)W$ wherein $R_w$ is hydrogen or $C_{1-6}$ alkyl and W is O or a bond; or

$R_1$ is a $C_{3-8}$ cycloalkyl group or a $C_{1-6}$ alkyl group optionally substituted by a group which is hydroxy, $C_{1-6}$ alkoxy, amino optionally substituted by one or two $C_{1-6}$ alkyl groups, $C_{1-7}$ alkanoylamino, $C_{3-8}$ cycloalkyloxy or $C_{3-8}$ cycloalkylamino; and $R_2$ is hydrogen; or

one of $R_1$ and $R_2$ is nitro, cyano or $C_{1-3}$ alkylcarbonyl and the other is a different group selected from nitro,

cyano, halo, $C_{1-3}$ alkylcarbonyl, methoxy or amino optionally substituted by one or two $C_{1-6}$ alkyl or by $C_{2-7}$ alkanoyl; or

$R_1$ and $R_2$ together with the carbon atoms to which they are attached, form 2,1,3-oxadiazole;

either one of $R_3$ and $R_4$ is hydrogen or $C_{1-4}$ alkyl and the other is $C_{1-4}$ alkyl; or

$R_3$ and $R_4$ together are $C_{2-5}$ polymethylene;

either $R_5$ is hydrogen, hydroxy, $C_{1-6}$ alkoxy, $C_{1-7}$ acyloxy or $ONO_2$; and

$R_6$ is hydrogen; or

$R_5$ and $R_6$ together are a bond;

J is hydrogen or $C_{1-6}$ alkyl;

E is a group of structure i):

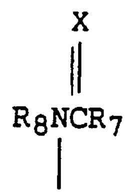

i)

wherein

either X is oxygen or sulphur; and

$R_7$ is hydrogen, $C_{1-6}$ alkyl optionally substituted by hydroxy, $C_{1-6}$ alkoxy, $C_{1-6}$ alkoxycarbonyl or carboxy, $C_{1-6}$ alkyl substituted by halogen, or $C_{2-6}$ alkenyl; aryl or heteroaryl either being optionally substituted by one or more groups or atoms selected from the class of $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and

$R_8$ is hydrogen, $C_{1-6}$ alkyl, or a group $OR_p$ or $NHCOR_q$ wherein $R_p$ is hydrogen, $C_{1-6}$ alkyl, aralkyl, $C_{1-7}$ alkanoyl or aroyl and $R_q$ is as defined above for $R_7$; or

$R_7$ and $R_8$ are joined together to form $C_{3-4}$ polymethylene optionally substituted by one or two $C_{1-6}$ alkyl groups or $-CH_2-(CH_2)_n-Z-(CH_2)_m-$wherein m and n are integers 0 to 2 such that m+n is 1 or 2 and Z is oxygen, sulphur or $NR_9$ wherein $R_9$ is hydrogen, $C_{1-9}$ alkyl, $C_{2-7}$ alkanoyl, phenyl $C_{1-4}$ alkyl, naphthylcarbonyl, phenylcarbonyl or benzylcarbonyl optionally substituted in the phenyl or naphthyl ring by one or two $C_{1-6}$ alkyl, $C_{1-6}$ alkoxy or halogen; mono- or bi-cyclic heteroarylcarbonyl; or $R_7$ and $R_8$ are joined to form $-B^1=B^2-B^3=B^4-$ wherein one of $B^1$ to $B^4$ is $CR_r$ or N and the other three are $CR_r$ wherein $R_r$ is hydrogen or $C_{1-6}$ alkyl;

or X is N-CN, $N-NO_2$, $N-COR_{10}$ or $N-SO_2R_{10}$ wherein $R_{10}$ is $C_{1-3}$ alkyl, $NH_2$, $NH(C_{1-3}$ alkyl), $CF_3$ or phenyl optionally substituted as defined for $R_x$; and

$R_7$ is $NHR_{11}$ wherein $R_{11}$ is hydrogen, $C_{1-6}$ alkyl, $C_{3-6}$ cycloalkyl, $C_{2-6}$ alkenyl or $C_{2-6}$ alkynyl; and

$R_8$ is hydrogen or $C_{1-6}$ alkyl; or

$R_7$ and $R_8$ together are $C_{2-4}$ polymethylene;

or E is a group of structure ii):

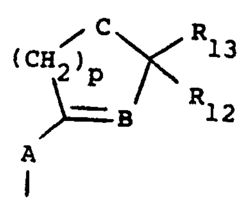

ii)

wherein

A is O or $NR_{14}$ wherein $R_{14}$ is hydrogen, $C_{1-4}$ alkyl, formyl, acetyl or hydroxymethyl;

B is N or $CR_{15}$ wherein $R_{15}$ is hydrogen, halogen, formyl or hydroxymethyl;

C is $CH_2$, O, S, CH-Halogen, amino or $C_{1-6}$ alkylamino;

p is 1, 2 or 3; and

$R_{12}$ and $R_{13}$ are independently hydrogen or methyl or together are oxo- or thio-;

or E is tetrahydroisoquinolinone, 2,3-dihydro-1H-isoindol-1-one, 2-pyridine N-oxide, 2-hydroxyphenyl or 2-hydroxypyridine (all four possible isomers);

the E moiety being trans to the $R_5$ group when $R_5$ is other than hydrogen or a bond.

3. A use according to claim 2 wherein the compound of formula (I) is wherein a and b together form an -O-linkage, E is $R_8NCXR_7$ wherein X is oxygen or sulphur, J is hydrogen, and:-
either one of $R_1$ and $R_2$ is hydrogen and the other is selected from the class of $C_{1-6}$ alkylcarbonyl, $C_{1-6}$ alkoxycarbonyl, $C_{1-6}$ alkylcarbonyloxy, $C_{1-6}$ alkylhydroxymethyl, nitro, cyano, chloro, trifluoromethyl, $C_{1-6}$ alkylsulphinyl, $C_{1-6}$ alkylsulphonyl, $C_{1-6}$ alkoxysulphinyl, $C_{1-6}$ alkoxysulphonyl, $C_{1-6}$ alkylcarbonylamino, $C_{1-6}$ alkoxycarbonylamino, $C_{1-6}$ alkyl-thiocarbonyl, $C_{1-6}$ alkoxy-thiocarbonyl, $C_{1-6}$ alkylthiocarbonyloxy, 1-mercapto $C_{2-7}$ alkyl, formyl, or aminosulphinyl, aminosulphonyl or aminocarbonyl, the amino moiety being optionally substituted by one or two $C_{1-6}$ alkyl groups, or $C_{1-6}$ alkylsulphinylamino, $C_{1-6}$ alkylsulphonylamino, $C_{1-6}$ alkoxysulphinylamino or $C_{1-6}$ alkoxysulphonylamino, or ethylenyl terminally substituted by $C_{1-6}$ alkylcarbonyl, nitro or cyano, or -C($C_{1-6}$ alkyl)NOH or -C($C_{1-6}$ alkyl)$NNH_2$; and
$R_7$ is hydrogen, $C_{1-6}$ alkyl optionally substituted by hydroxy, $C_{1-6}$ alkoxy, $C_{1-6}$ alkoxycarbonyl or carboxy, $C_{1-6}$ alkyl substituted by halogen, or $C_{2-6}$ alkenyl; aryl or heteroaryl either being optionally substituted by one or more groups or atoms selected from the class of $C_{1-6}$ alkoxy, hydroxy, halogen, trifluoromethyl, nitro, cyano, $C_{1-12}$ carboxylic acyl, or amino or aminocarbonyl optionally substituted by one or two $C_{1-6}$ alkyl groups; and
$R_8$ is hydrogen or $C_{1-6}$ alkyl; or
$R_7$ and $R_8$ are joined together to form $C_{3-4}$ polymethylene or $-CH_2-(CH_2)_n-Z-(CH_2)m-$ wherein m and n are integers 0 to 2 such that m+n is 1 or 2 and Z is oxygen, sulphur or $NR_9$ wherein $R_9$ is hydrogen, $C_{1-9}$ alkyl, $C_{2-7}$ alkanoyl, phenyl $C_{1-4}$ alkyl, naphthylcarbonyl, phenylcarbonyl or benzylcarbonyl optionally substituted in the phenyl or naphthyl ring by one or two $C_{1-6}$ alkyl, $C_{1-6}$ alkoxy or halogen; mono- or bicyclic heteroarylcarbonyl;
and the remaining variables are as defined in claim 2.

4. A use according to claim 3 wherein Y is N or C-$R_1$ wherein $R_1$ is $C_{1-6}$ alkyl, $CF_3$, nitro, cyano or $C_{1-3}$ alkylcarbonyl, and $R_2$ is hydrogen.

5. A use according to claim 3 wherein $R_3$ and $R_4$ are both methyl groups.

6. A use according to claim 3 wherein $R_7$ and $R_8$ are joined together to form $C_{3-4}$ polymethylene or $-CH_2-(CH_2)_n-Z-(CH_2)_m-$ wherein n, m and Z are as defined in claim 2.

7. A use according to claim 3 wherein $R_8$ is hydrogen or methyl and $R_7$ is methyl, ethyl, hydrogen, or phenyl optionally substituted by one, two, three or four groups or atoms selected from methyl, methoxy, hydroxy, fluoro and chloro.

8. A use according to claim 6 wherein the compound of formula (I) is (±)-6-cyano3,4-dihydro-2,2-dimethyl-trans-4-(2-oxo-1-pyrrolidinyl)- 2H-benzo[b]pyran-3-ol, or the pure (-)-enantiomer thereof.

9. A use according to claim 1 wherein the potassium channel activator is in a 0.1 to 50mg unit dose.